# FASCICULE DE BREVET EUROPEEN

(11) **EP 3 423 422 B1**
(45) Date de publication et mention de la délivrance du brevet: **05.06.2024**
(21) Numéro de dépôt: 17725697.1
(22) Date de dépôt: 28.04.2017
(51) Int. Cl.: C04B 35/111, C04B 35/64, C04B 41/00, C04B 41/48, C04B 41/83, C04B 38/00, A61K 6/17, A61K 6/802, A61C 13/083, A61C 13/00, C04B 111/00

(54) **PRÉFORME DESTINÉE À LA FABRICATION D'UNE PROTHÈSE DENTAIRE**
VORFORM ZUR HERSTELLUNG EINES ZAHNERSATZTEILS
PREFORM FOR THE PRODUCTION OF A DENTAL PROSTHESIS

(30) Priorité: 04.03.2016 FR 1651840
(43) Date de publication de la demande: 09.01.2019
(73) Titulaire: VITA-ZAHNFABRIK H. Rauter GmbH & Co. KG, 79713 Bad Säckingen (DE)
(72) Inventeur: Sadoun, Michael, 4130 Esneux (BE)
(74) Mandataire: Cabinet Nony
(86) Numéro de dépôt international: PCT/FR2017/051027
(87) Numéro de publication internationale: WO 2017/149262

(56) Documents cités:
- WO-A1-2015/168463
- FR-A1- 2 935 897
- US-A- 5 843 348
- CHUN HONG CHEN ET AL: "Fabrication and Characterization of Porous Alumina Tube with Pore Gradient", MATERIALS SCIENCE FORUM, vol. 492-493, 15 août 2005 (2005-08-15), pages 755-760, XP055316988, DOI: 10.4028/www.scientific.net/MSF.492-493.755 cité dans la demande
- FIGIEL PAWEL ET AL: "Al2O3and ZrO2powders formed by centrifugal compaction using the ultra HCP method", CERAMICS INTERNATIONAL, vol. 39, no. 1, 2013, pages 635-640, XP028955615, ISSN: 0272-8842, DOI: 10.1016/J.CERAMINT.2012.06.075 cité dans la demande

## Description

### Domaine technique

L'invention se rapporte à une préforme, à un support poreux obtenu par frittage d'une telle préforme, à un bloc composite obtenu par imprégnation d'un tel support poreux au moyen d'une résine, et à une prothèse dentaire fabriquée à partir d'un tel bloc composite. L'invention se rapporte encore à un procédé de fabrication d'une telle préforme, d'un tel support poreux et d'un tel bloc composite.

### État de la technique

On connaît des blocs composites comportant un support, généralement en matière céramique, et une résine remplissant, au moins partiellement, les interstices du support. Classiquement, le support poreux, avant infiltration de la résine, est obtenu par frittage d'une préforme.

Pour fabriquer le bloc composite, de la résine à l'état liquide est infiltrée dans les pores accessibles, ou « ouverts », du support, généralement par capillarité. Après durcissement de la résine, le bloc composite obtenu est usiné à la forme définitive souhaitée, classiquement par « conception et usinage assistés par ordinateur » ou CAD-CAM (« Computer Aided Design - Computer Aided Machining »).

Des procédés de fabrication de blocs composites sont notamment décrits dans les documents suivants : US 5,869,548, US 5,843,348, US 5,250,352, EP 0 241 384, WO93/07846, EP 2 725 358, EP 0 240 643, FR 2 904 306, EP 0 701 808 ou US 7,294,392.

WO2010029515 décrit encore un bloc composite destiné à la fabrication d'une prothèse dentaire.

US 13/063,365 décrit un procédé d'infiltration, sous haute pression, d'un support poreux au moyen d'une résine.

Par ailleurs, le Rapport de Recherche de la demande française 16 51840 cite plusieurs documents :
L'article *«* Fabrication and Characterization of Porous Alumina Tube with Pore Gradient » de C.H. CHEN et al., dans « Materials Science Forum » 492-493, pp. 755-760, décrit la fabrication de tubes poreux par centrifugation d'une poudre dont la fraction solide, unimodale, présente une taille de particules moyenne de 0,5 µm, et de particules d'un agent porogène. La centrifugation a pour objectif de modifier la porosité, et non pas la distribution de la taille des grains.

US 5,843,348 décrit un procédé dans lequel une suspension peut être mise en forme par centrifugation. Cette centrifugation est une alternative à une mise en forme par pression. La centrifugation est donc utilisée comme moyen de compaction, et non pas comme moyen pour créer un gradient de propriétés de la structure. En outre, ce document ne suggère pas une distribution bimodale des tailles de particules de la suspension pour obtenir un gradient dans les tailles de particules.

L'article intitulé « Al2O3 and ZrO2 powders formed by centrifugal compaction using the ultra HCP method », par P. FIGIEL et al., dans « Ceramics international » 39 (2013) 635-640, s'intéresse à l'influence de la compaction par centrifugation sur des produits frittés obtenus par centrifugation de suspensions de microparticules d'Al₂O₃ et de Z₁O₂. Les mélanges de particules d'Al₂O₃ et de ZrO₂ conduisent à des produits frittés opaques, noirs, qui ne sont pas adaptés à la fabrication de prothèses dentaires. En outre, D3 cherche à obtenir un produit fritté homogène, et donc à éviter tout gradient dans les propriétés mécaniques et optiques.

Les blocs composites fabriqués selon les procédés actuels ne permettent pas de fabriquer des prothèses dentaires présentant des propriétés optiques et mécaniques correspondant précisément à celles d'une dent naturelle, ce qui limite l'exploitation commerciale de ces blocs composites.

Il existe donc un besoin pour des blocs composites permettant de fabriquer des prothèses présentant des propriétés optiques et mécaniques correspondant précisément à celles d'une dent naturelle. Il existe également un besoin permanent pour prolonger la durée de vie des prothèses obtenues à partir de blocs composites.

Un but de l'invention est de répondre, au moins partiellement, à ces besoins.

### Résumé de l'invention

Selon l'invention, on atteint ce but au moyen d'une préforme comportant, de préférence étant constituée, d'un ensemble de particules agglomérées, en matière céramique, en matière vitrocéramique ou en verre, tel que, en pourcentages volumiques
- plus de 40%, de préférence plus de 50%, de préférence plus de 60%, et moins de 90% des particules dudit ensemble présentent une taille supérieure à 0,5 µm, de préférence supérieure à 1,0 µm, de préférence supérieure à 1,5 µm, de préférence supérieure à 2,0 \µm, et inférieure à 3,5 µm, de préférence inférieure à 3,0 µm, lesdites particules étant désignées ci-après « particules d'émail », et
- plus de 10%, de préférence plus de 20%, de préférence plus de 30%, et moins de 60% des particules dudit ensemble présentent une taille supérieure à 3,5 µm, de préférence supérieure à 4,0 µm, et inférieure à 5,5 µm, de préférence inférieure à 5,0 µm, lesdites particules étant désignées ci-après « particules de dentine »,

la microstructure de la préforme étant telle qu'il existe un axe X, dit « axe de variation », suivant lequel le rapport Ve/(Ve+Vd) , ou « densité locale », évolue de manière continue, Ve et Vd désignant les pourcentages volumiques en particules d'émail et en particules de dentine, respectivement,
les particules d'émail et de dentine représentant, ensemble, plus de 90% du volume des particules agglomérées, la distribution granulométrique de l'ensemble des particules étant bimodale et comportant des premier et deuxième modes principaux, le premier mode principal étant supérieur à 1,5 µm et inférieur à 3,5 µm, le deuxième mode principal étant supérieur à 3,5 µm et inférieur à 5,5 µm.

Comme on le verra plus en détail dans la suite de la description, la variation continue du rapport Ve/(Ve+Vd) supprime toute trace de strate, ce qui permet de fabriquer un support poreux présentant des variations progressives de teintes et/ou de propriétés mécaniques. Avantageusement, la prothèse dentaire obtenue à partir d'un tel support se présente sans ligne d'interface entre différentes régions de la prothèse.

La variation du rapport Ve/(Ve+Vd) exprime la présence d'un gradient dans les tailles des particules. Un tel gradient ne correspond pas à un gradient dans la taille des pores, ni dans la quantité de pores.

En particulier, lorsque la préforme est fabriquée par centrifugation d'une suspension comportant des particules et un solvant, comme décrit ci-après, les caractéristiques de la porosité dépendent de nombreux paramètres, comme la forme des particules, la distribution granulométrique des particules, la densité du matériau constitutif des particules, les propriétés de surface des particules, et en particulier le potentiel zéta, le pH du solvant, l'intensité de la centrifugation, le temps de centrifugation... On ne saurait donc déduire des caractéristiques relatives à la distribution des tailles de particules à partir de caractéristiques relatives à la distribution des tailles de pores ou relatives à la quantité de pores.

Une préforme selon l'invention peut encore comporter une ou plusieurs des caractéristiques optionnelles suivantes :
- les particules d'émail présentent une taille moyenne D₅₀ supérieure à 1,5 µm et inférieure à 3,0 µm, et/ou les particules de dentine présentent une taille moyenne D₅₀ supérieure à 4,0 µm et inférieure à 5,0 µm ;
- suivant l'axe de variation, le pourcentage volumique de particules d'émail varie de manière opposée, de préférence de manière complémentaire, au pourcentage volumique de particules de dentine ;
- suivant l'axe de variation, la concentration des particules d'émail et des particules de dentine, c'est-à-dire le volume total des particules d'émail et des particules de dentine par unité de volume de la préforme, présente une variation inférieure à 20%, de préférence inférieure à 10% par rapport à sa valeur minimale le long dudit axe de variation, l'unité de volume étant un volume de 1 mm³ ;
- la préforme présente une première région, dite « région d'émail », dans laquelle le rapport Ve / (Ve+Vd) est supérieur à 0,6, de préférence supérieur à 0,7, de préférence supérieur à 0,8, de préférence supérieur à 0,9, et une deuxième région, dite « région de dentine », dans laquelle le rapport Ve / (Ve+Vd) est inférieur à 0,5, de préférence inférieur à 0,4, de préférence inférieur à 0,3, de préférence inférieur à 0,2, de préférence inférieur à 0,05, les régions d'émail et de dentine étant de préférence sous la forme de couches et s'étendant de préférence à partir de faces d'émail et de dentine opposées de la préforme, en considérant l'axe de variation ;
- les particules d'émail et de dentine représentent ensemble plus de 95%, de préférence plus de 98%, de préférence sensiblement 100% du volume de la masse dudit ensemble de particules ;
- plus de 90%, de préférence plus de 95%, de préférence plus de 98% de l'ensemble constitué par les particules d'émail et les particules de dentine, en pourcentage en nombre, sont en un matériau présentant un indice de réfraction supérieur à 1,40, de préférence supérieur à 1,45 et/ou inférieur à 1,70, de préférence inférieur à 1,65 ;
- de préférence, la densité ρₑ des particules d'émail est sensiblement identique à la densité ρ_{d} des particules de dentine ;
- de préférence, le rapport ρₑ/ ρ_{d} est supérieur à 0,9, de préférence supérieur à 0,95, de préférence supérieur à 0,98 et inférieur à 1,10, de préférence inférieur à 1,05, de préférence inférieur à 1,02 ;
- de préférence, l'ensemble constitué par les particules d'émail et les particules de dentine comporte moins de 1%, de préférence moins de 0,5%, de préférence moins de 0,1%, en pourcentage massique, de zircone, de préférence ne comporte pas de zircone.

L'invention concerne également un procédé de fabrication d'une préforme selon l'invention, ledit procédé comportant les étapes suivantes :
A) préparation d'une suspension comportant, de préférence consistant en :
   - un ensemble de particules, ou « charge particulaire », de préférence en matière céramique, vitrocéramique ou verre, ledit ensemble comportant, en pourcentages volumiques sur la base du volume de la masse dudit ensemble de particules :
      - plus de 30%, de préférence plus de 40%, et moins de 70% de particules d'émail, et
      - plus de 30%, de préférence plus de 40% et moins de 70% de particules de dentine,

   - un solvant ;
B) modification de la distribution spatiale des particules de la suspension, de préférence par centrifugation de la suspension ;
C) consolidation des particules de manière à former une préforme.

Comme on le verra plus en détail dans la suite de la description, la modification de la distribution spatiale des particules de la suspension, rendue possible par l'utilisation d'une fraction solide bimodale spécifique, permet d'adapter localement le rapport Ve/(Ve+Vd), et ainsi d'adapter l'aspect, mais aussi les propriétés mécaniques locales, de la préforme et en conséquence du support poreux fritté, du bloc composite et de la prothèse.

L'invention concerne également un procédé de fabrication d'un support poreux, ledit procédé comportant une fabrication d'une préforme selon l'invention, puis une étape D) de frittage de ladite préforme, l'intensité du frittage étant variable en fonction de la région de la préforme considérée.

Un tel procédé peut encore comporter une ou plusieurs des caractéristiques optionnelles suivantes :
- l'intensité du frittage d'une région de la préforme est variable en fonction de sa position le long de l'axe de variation ;
- l'étape D) comporte
   - un frittage de base, de préférence effectué pendant une durée supérieure à 1 h et inférieure à 4 h, lors duquel toute la surface extérieure de la préforme reçoit sensiblement la même densité de flux calorifique (frittage homogène) ; et
   - un frittage additionnel, de préférence effectué à une température supérieure de plus de 30°C, de plus de 50°C, voire de plus de 100°C, de plus de 150°C ou de plus de 200°C à la température du frittage de base, pendant une durée de préférence supérieure à 15 min et de préférence inférieure à 4 h, lors duquel la densité de flux thermique est variable en fonction de la partie de la surface extérieure de la préforme considérée.
- lors du frittage additionnel, la densité de flux thermique, c'est-à-dire l'intensité du frittage, est d'autant plus élevée que le rapport Ve/(Ve+Vd) est élevé ;
- lors du frittage additionnel, une face de la préforme à proximité de laquelle la concentration en particules d'émail est la plus élevée, dite « face d'émail », repose sur une plaque chauffante.

L'invention concerne encore un support poreux fabriqué suivant un procédé selon l'invention, ledit support comportant une région présentant une porosité ouverte comprise entre 5% à 20%, dite « région poreuse », et une région présentant une porosité ouverte supérieure à 20% et inférieure à 40%, dite « région très poreuse », la région poreuse présentant une porosité ouverte inférieure de 5% à la région très poreuse, lesdites régions poreuse et très poreuse présentant chacune un volume supérieur à 30 mm³, de préférence supérieur à 50 mm³, de préférence supérieur à 100 mm³, de préférence supérieur à 150 mm³.

En mesurant l'épaisseur suivant l'axe de variation X, lesdites régions poreuse et très poreuse présentent la forme de couches d'épaisseur supérieure à 1 mm, de préférence supérieure à 3 mm, de préférence supérieure à 5 mm.

L'invention concerne aussi un procédé de fabrication d'un bloc composite, ledit procédé comportant une fabrication d'un support poreux selon un procédé selon l'invention, puis des étapes E) et F) suivantes :
E) infiltration du support poreux au moyen d'une résine à l'état liquide ;
F) durcissement de toute la résine à l'état liquide imprégnant le support ;

Les étapes E) et F) étant effectuées sous une pression de préférence supérieure à 1000 bar.

L'invention concerne enfin un bloc composite, en particulier fabriqué suivant un procédé de fabrication selon l'invention, le bloc composite comportant une région dite « très dure » présentant une dureté supérieure à 240 Vickers, et de préférence un module élastique de Young, mesuré suivant la norme ISO 10 477, supérieur à 30 GPa, et une région dite « dure » présentant une dureté supérieure à 60 Vickers et inférieure à 180 Vickers, de préférence inférieure à 170 Vickers, voire inférieure à 160 Vickers, et, de préférence, un module élastique de Young, mesuré suivant la norme ISO 10 477, supérieur à 15 GPa et inférieur à 30 GPa.

Lesdites régions très dure et dure correspondent de préférence à des régions poreuse et très poreuse, respectivement, du support poreux d'origine. Elles présentent chacune un volume supérieur à 30 mm³, de préférence supérieur à 50 mm³, de préférence supérieur à 100 mm³, de préférence supérieur à 150 mm³.

En mesurant l'épaisseur suivant l'axe de variation X, lesdites régions très dure et dure présentent la forme de couches d'épaisseur supérieure à 1 mm, de préférence supérieure à 3 mm, de préférence supérieure à 5 mm.

### Brève description des figures

D'autres caractéristiques et avantages de l'invention apparaitront encore à la lecture de la description détaillée qui va suivre et à l'examen du dessin annexé, fournis à des fins illustratives et non limitatives, dans lequel :
- la figure 1 illustre un procédé de fabrication d'une prothèse dentaire selon l'invention ;
- les figures 2 et 3, et 4 illustrent très schématiquement les étapes B) et D), respectivement, d'un procédé de fabrication d'une prothèse dentaire selon l'invention ;
- la figure 5 représente très schématiquement un bloc composite dans un mode de réalisation préféré de l'invention .

### Définitions

Une « préforme » est classiquement une masse solide à porosité ouverte constituée de particules agglomérées, c'est-à-dire solidarisées les unes aux autres sans frittage ni fusion de ces particules. Cette agglomération peut résulter, notamment, d'une compaction des particules, de préférence sans déformation plastique des particules, ou d'un mélange des particules avec un liant ou sans liant.
- Par « prothèse dentaire », on entend de manière générale toute pièce destinée à être placée sur la denture d'un patient dans le but de la restaurer en totalité ou en partie dans sa forme naturelle et sa fonction.

C'est ainsi que les prothèses dentaires fabriquées selon l'invention peuvent être, par exemple, des chapes périphériques ou couronnes qui viennent se placer sur le moignon d'une dent naturelle, ou bien des prothèses généralement désignées sous les termes de « inlay » et « onlay » qui sont destinées à reconstituer une altération partielle d'une dent en remplissant la cavité résultant de la perte de substance de la dent par une pièce de même forme réalisée par le prothésiste, ou bien encore des bridges qui sont des prothèses qui prennent appui simultanément sur les parties subsistantes d'au moins deux dents en compensant éventuellement une ou plusieurs des dents manquantes, ou encore des couronnes dentaires vissées sur des implants. En fonction de la nature de la prothèse dentaire fabriquée, un bloc composite selon l'invention peut être rendu solidaire d'autres pièces, par exemple d'une base métallique.
- La « taille » d'une particule d'une poudre est donnée classiquement par une caractérisation de distribution granulométrique. Un granulomètre laser permet la mesure des tailles inférieures ou égale à 5 mm.

Les percentiles ou « centiles » 10 (D₁₀), 50 (D₅₀), 90 (D₉₀) et 99,5 (D_{99,5}) d'une poudre sont les tailles de particules correspondant aux pourcentages, en masse, de 10 %, 50 %, 90 % et 99,5 % respectivement, sur la courbe de distribution granulométrique cumulée des particules de la poudre, les tailles de particules étant classées par ordre croissant. Par exemple, 10 %, en masse, des particules de la poudre ont une taille inférieure à D₁₀ et 90 % des particules en masse ont une taille supérieure à D₁₀. Les percentiles peuvent être déterminés à l'aide d'une distribution granulométrique réalisée à l'aide d'un granulomètre laser.

On appelle « taille maximale » le percentile 99,5 (D_{99,5}) de ladite poudre.

On appelle « taille médiane » le percentile D₅₀, c'est-à-dire la taille divisant les particules en première et deuxième populations égales en masse, ces première et deuxième populations ne comportant que des particules présentant une taille supérieure, ou inférieure respectivement, à la taille médiane.

Dans une préforme, les particules ne sont plus sous la forme d'une poudre, mais sont agglomérées, par compaction ou au moyen d'un liant, de préférence d'un liant temporaire. Leurs dimensions sont cependant les mêmes que celles qu'elles avaient dans la charge de départ ayant été préparée pour former la préforme. La taille des particules au sein de la préforme peut donc être évaluée à partir des caractéristiques des poudres utilisées pour constituer la charge de départ. La taille des particules dans la préforme peut être également classiquement évaluée par des analyses d'images de coupes de la préforme. Ces images peuvent être en particulier obtenues par microscopie électronique à balayage (SEM).

Le frittage de la préforme conduit à un support poreux. Lors du frittage, il se forme des cous de frittage et les particules se fixent solidement les unes aux autres. Leurs dimensions ne sont cependant pas substantiellement modifiées. Des analyses d'images de coupes du support permettent donc également d'évaluer la distribution granulométrique des particules de la préforme.
- La taille moyenne des pores peut être classiquement mesurée avec un porosimètre à mercure.
- Sauf indication contraire, « comportant un », « comprenant un » ou « présentant un » doivent être interprétés de manière non exclusive.
- Les pourcentages volumiques relatifs à des particules, par exemple les pourcentages en particules d'émail Ve et en particules de dentine Vd, sont des pourcentages sur la base de la masse de ces particules, c'est-à-dire en ignorant les interstices entre les particules.
- 1 bar est égal à 0,1 MPa.

### Description détaillée

### Préforme

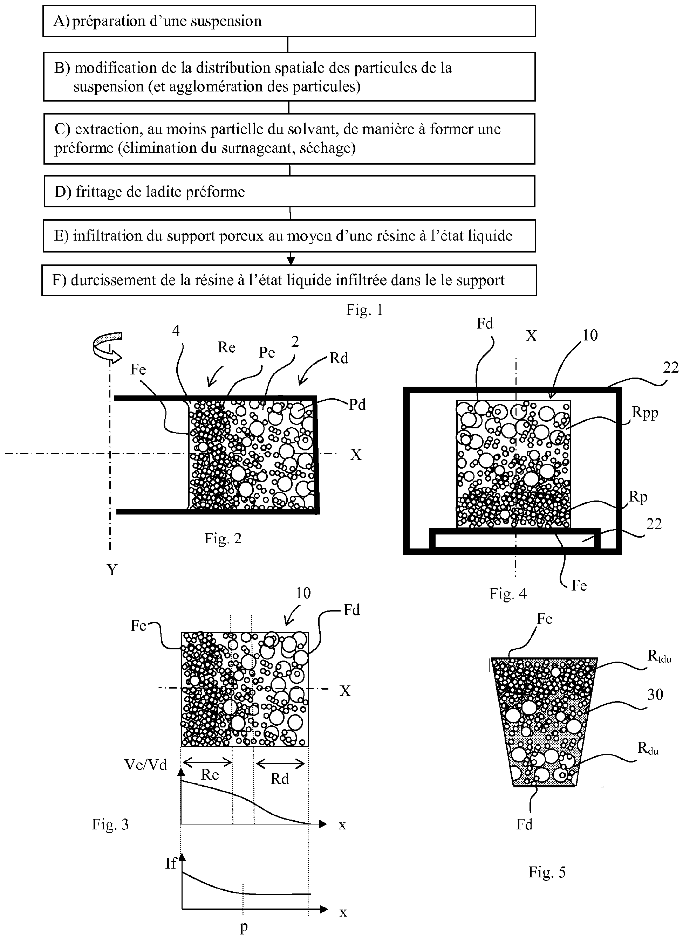

Comme représenté sur la figure 3, une préforme 10 selon l'invention est constituée d'un ensemble de particules agglomérées.

Le matériau constitutif des particules peut être tout matériau couramment utilisé pour la fabrication de prothèses dentaires.

De préférence, plus de 50%, plus de 70%, plus de 90%, de préférence plus de 95%, de préférence plus de 98%, de préférence 100% en volume des particules dudit ensemble sont un même matériau, de préférence céramique.

De préférence, le matériau est en un ou plusieurs oxydes métalliques, sous forme de vitrocéramique, de verre, ou de céramique cristalline telle que le quartz, l'alumine ou la mullite.

De préférence, la taille maximale de l'ensemble de particules est supérieure à 1 µm et/ou inférieure à 10 µm.

De préférence, la taille minimale de l'ensemble de particules est supérieure à 0,01µm et/ou inférieure à 0,5 µm.

De préférence, la taille médiane de l'ensemble de particules est supérieure à 1 µm et/ou inférieure à 10 µm.

Selon l'invention, la distribution spatiale des particules dépend de leurs tailles. En particulier, il existe des régions dans lesquelles les pourcentages volumiques des particules d'émail Pe, c'est-à-dire des particules très fines présentant une taille supérieure à 1,5 µm et inférieure à 3,5 µm, sont différents.

Il existe également des régions dans lesquelles les pourcentages volumiques des particules de dentine Pd, c'est-à-dire des particules fines présentant une taille supérieure à 3,5 µm et inférieure à 5,5 µm, sont différents.

Le pourcentage volumique peut être évalué en divisant le volume occupé par les particules considérées par le volume de la région considérée. La région peut être par exemple une région cubique de 1 mm de côté.

De préférence, suivant l'axe de variation X, le pourcentage volumique de particules d'émail varie de manière inverse, de préférence de manière inversement proportionnelle au pourcentage volumique de particules de dentine, c'est-à-dire qu'une région comporte d'autant plus de particules d'émail qu'elle comporte moins de particules de dentine, comme représenté sur la figure 3.

La distribution granulométrique (nombre de particules en fonction de la taille des particules) de l'ensemble de particules est bimodale, c'est-à-dire comporte des premier et deuxième modes principaux, le premier mode principal étant supérieur à 1,5 µm, de préférence supérieur à 2,0 µm, et inférieur à 3,5 µm, de préférence inférieur à 3,0 µm, le deuxième mode étant supérieur à 3,5 µm, de préférence supérieur à 4,0 µm, et inférieur à 5,5 µm, de préférence inférieur à 5,0 µm.

Le rapport Ve/(Ve+Vd) évolue de manière continue suivant un axe X, dit « axe de variation ».

L'axe de variation peut être rectiligne ou non. De préférence, il est rectiligne.

Une évolution « continue » correspond à une évolution telle que, le long de l'axe de variation, il n'existe pas de palier pour le rapport Ve/(Ve+Vd), sauf éventuellement dans les parties régions extrêmes de la préforme.

De préférence, au sein de la préforme, il n'existe pas de palier pour le rapport Ve/(Ve+Vd), qui évolue donc constamment le long de l'axe de variation.

De préférence, comme représenté sur la figure 3, au sein de la préforme, l'évolution du rapport Ve/(Ve+Vd) est monotone, c'est-à-dire que ce rapport est toujours croissant ou toujours décroissant, le long de l'axe d'hétérogénéité.

De préférence, au sein de la préforme, la préforme présente une première région, dite « région d'émail », dans laquelle le rapport Ve/(Ve+Vd) est supérieur à 0,9, et une deuxième région, dite « région de dentine », dans laquelle le rapport Ve/(Ve+Vd) est inférieur à 0,05.

De préférence, au sein de la préforme, chacune des régions d'émail et de dentine a un volume supérieur à 30 mm³, de préférence supérieur à 50 mm³, de préférence supérieur à 100 mm³, de préférence supérieur à 150 mm³.

De préférence, le rapport Ve/(Ve+Vd) évolue de manière identique le long de n'importe quelle ligne parallèle à l'axe de variation. Ainsi, dans une tranche de la préforme perpendiculaire à l'axe de variation, d'épaisseur très faible, le rapport Ve/(Ve+Vd) est sensiblement constant.

De préférence, les régions d'émail et de dentine sont donc sous la forme de couches, s'étendant de préférence à partir de faces d'émail Fe et de dentine Fd opposées de la préforme, de préférence sensiblement perpendiculairement à l'axe de variation.

De préférence, chacune desdites couches a une épaisseur supérieure à 1 mm, de préférence supérieure à 2 mm, de préférence supérieure à 3 mm, de préférence supérieure à 4 mm, de préférence supérieure à 5 mm.

De préférence, il existe au moins une région d'émail et une région de dentine occupant, ensemble, plus de 70%, plus de 80%, plus de 90%, de préférence 100% du volume de la préforme.

### Procédé de fabrication de la préforme

Un procédé comportant les étapes A) à C) est bien adapté pour fabriquer une préforme selon l'invention.

**A l'étape A),** une suspension est classiquement préparée en mélangeant dans un récipient des poudres dans un solvant 4.

La fraction solide de la suspension représente de préférence plus de 50%, de préférence plus de 60%, en volume, et moins de 75%, de préférence moins de 70% en volume de la suspension.

De préférence, on mélange une première poudre de particules Pe présentant une taille médiane supérieure à 1,5 µm, de préférence supérieure à 2,0 µm, et inférieure à 3,5 µm, de préférence inférieure à 3,0 µm ou "poudre d'émail", et une deuxième poudre de particules Pd présentant une taille médiane supérieure à 3,5 µm, de préférence supérieure à 4,0 µm, et inférieure à 5,5 µm, de préférence inférieure à 5,0 µm ou "poudre de dentine". De préférence, les première et deuxième poudres représentent ensemble plus de 90%, plus de 95%, de préférence 100% de la masse de la fraction solide.

De préférence, la fraction solide est constituée des poudres d'émail et de dentine.

De préférence, la fraction solide ne comporte pas d'agent porogène. Avantageusement, les propriétés mécaniques en sont améliorées.

De préférence, la fraction solide est constituée, pour plus de 90%, plus de 95%, de préférence 100% de sa masse, de particules en un ou plusieurs oxydes métalliques, sous forme de vitrocéramique, de verre, ou de céramique cristalline telle que le quartz, l'alumine ou la mullite.

Dans un mode de réalisation, les particules de dentine contiennent des pigments colorés, en particulier des pigments colorés classiquement utilisés pour la fabrication de prothèses dentaires.

Dans un mode de réalisation, les particules d'émail ne contiennent pas de pigments colorés.

La seule variation de la distribution spatiale des particules permet en effet d'obtenir des variations de teintes correspondant aux variations naturelles des dents.

Le solvant est de préférence choisi dans le groupe constitué par l'eau et les mélanges eau + éthanol. Classiquement, il peut également contenir un agent modificateur de pH, par exemple de l'acide chlorhydrique et/ou de l'acide acétique, et/ou un défloculant, par exemple du sodium polyméthacrylate et/ou du silicate de sodium, et/ou du sodium polycarboxylate, et/ou un liant, par exemple de l'alcool polyvinylique. De préférence, le solvant ne comporte pas d'agent porogène, comme du PMMA.

**A l'étape B),** le récipient est de préférence mis en rotation, de préférence autour d'un axe de rotation Y, de manière à centrifuger la suspension et créer ainsi une ségrégation entre les particules.

Lors d'une centrifugation, comme représenté sur la figure 2, la distribution spatiale des particules de la suspension varie selon la direction de la force centrifuge. Lorsque toutes les particules sont dans le même matériau et présentent des formes et des densités similaires, la concentration en grosses particules augmente à mesure que l'on s'éloigne du centre de rotation, suivant l'axe de variation X correspondant à la direction de la force centrifuge et donc perpendiculaire à l'axe de rotation Y.

La variation des conditions de centrifugation et de la viscosité de la suspension permet d'adapter la distribution spatiale des particules.

La centrifugation est un procédé connu pour fabriquer des préformes homogènes et compactes. La centrifugation est ainsi classiquement utilisée avec des suspensions dont la fraction solide est unimodale, afin d'éviter toute hétérogénéité.

Les inventeurs ont découvert que la centrifugation, appliquée à une suspension comportant une fraction solide bimodale spécifique, permet de créer une hétérogénéité dans la distribution des tailles de particules, et d'obtenir finalement un bloc composite présentant des propriétés mécaniques et d'aspect variables en fonction de la région considérée. Comme indiqué précédemment, cette hétérogénéité des tailles de particules n'implique pas une évolution de la porosité, ni même de la taille des pores.

Les conditions de centrifugation sont la vitesse de rotation et la durée de centrifugation. De manière bien connue, pour une même suspension, la ségrégation des particules augmente avec l'intensité de la centrifugation, c'est-à-dire avec la vitesse de rotation et la durée de centrifugation. L'effet de la centrifugation dépend, de manière connue, de la nature du solvant, et notamment de sa viscosité, mais aussi des paramètres des particules, et notamment de leur composition et de leur forme. De simples essais permettent de déterminer des conditions de centrifugation adaptées.

La face de la suspension qui est la plus proche de l'axe de rotation Y lors de la centrifugation est appelée « face d'émail » Fe. Lorsque les particules de la suspension ont sensiblement toutes la même densité, c'est à proximité de cette face que la concentration en particules de plus petites tailles est la plus élevée. En particulier, c'est à proximité de cette face que la concentration en particules d'émail est la plus élevée.

La centrifugation participe à l'agglomération des particules.

La centrifugation produit une accélération de préférence supérieure à 50 G, de préférence supérieure à 80 G, de préférence supérieure à 100 G, de préférence supérieure à 130 G, voire supérieure à 150 G.

La durée de centrifugation est de préférence supérieure à 10 min, de préférence supérieure à 20 min, voire supérieure à 30 min.

**A l'étape C),** le solvant est extrait de la suspension, ce qui permet de renforcer l'agglomération des particules.

De préférence, après la centrifugation, le surnageant est versé hors de la suspension. Il peut être également éliminé par chauffage sous vide.

Ensuite, on effectue un séchage pour éliminer le liquide entre les particules agglomérées. A l'issue de l'étape C), on obtient une préforme selon l'invention, comme représenté sur la figure 3.

### Procédé de fabrication d'un support poreux à partir de la préforme

L'invention concerne également un procédé de fabrication d'un support poreux comportant les étapes A) à C) de manière à fabriquer une préforme selon l'invention, puis une étape D) de frittage de ladite préforme.

Dans un mode de réalisation, la préforme subit un frittage de base, de préférence sensiblement homogène. La température de frittage dépend de la nature des particules. L'homme du métier sait adapter cette température en fonction de la nature des particules.

La durée du frittage de base est de préférence supérieure à 1 h, de préférence supérieure à 2 h, de préférence supérieure à 3 h, et/ou inférieure à 5 h, de préférence inférieure à 3 h, de préférence inférieure à 2,5 h, de préférence inférieure à 2,25 h.

Le frittage de base est de préférence sensiblement homogène, c'est-à-dire que les densités de flux thermiques (en W/m²) soient sensiblement les mêmes quelle que soit la partie de la surface extérieure de la préforme considérée.

Les rampes de montée et de descente en température peuvent être, par exemple, comprise entre 25°C et 300°C/heure.

Le four 20 utilisé pour le frittage de base peut être un four de frittage conventionnel.

Les conditions de frittage sont différentielles, c'est-à-dire qu'elles dépendent de la région de la préforme considérée. La variation des conditions de frittage permet d'adapter localement la densité du support.

A défaut de frittage différentiel, le support poreux permet de fabriquer un bloc composite qui présente un gradient de propriétés optiques, mais sensiblement sans gradient de propriétés mécaniques, les particules d'email étant les plus petites. Or les propriétés mécaniques d'une dent naturelle sont différentes selon la région considérée. En particulier l'émail et la dentine ne présentent pas les mêmes propriétés mécaniques.

Le frittage différentiel permet avantageusement d'adapter les propriétés mécaniques de différentes régions du bloc composite aux régions correspondantes de la dent naturelle. En particulier, de préférence, le frittage est renforcé dans la région d'émail afin de réduire la porosité ouverte et augmenter la densité locale. Après imprégnation par la résine, la région d'émail du bloc composite présente avantageusement une dureté, un module élastique et une résistance à l'usure supérieures.

Les conditions de frittage sont la température de frittage et la durée de frittage, ou « pallier de frittage », c'est-à-dire la durée pendant laquelle la température de frittage est maintenue.

De manière bien connue, la densité d'une région du support augmente avec l'intensité du frittage, c'est-à-dire avec la température de frittage et la durée de frittage.

De préférence, l'intensité du frittage d'une première région, dite « région poreuse », est supérieure à l'intensité du frittage d'une deuxième région, dite « région très poreuse », comme représenté sur la figure 4.

La région poreuse comporte de préférence un rapport Ve/(Ve+Vd) supérieur au rapport Ve/(Ve+Vd) de la région très poreuse.

De préférence, la région poreuse subit, en plus du frittage de base, un frittage additionnel, que ne subit pas la région très poreuse. La durée du frittage additionnel est de préférence supérieure à 15 min, de préférence supérieure à 30 min, de préférence supérieure à 1 h, et/ou inférieure à 2 h, de préférence inférieure à 3 h, de préférence inférieure à 2,5 h, de préférence inférieure à 2,25 h.

De préférence, pour le frittage additionnel, la préforme est chauffée suivant une direction privilégiée, dite "direction de chauffage", c'est-à-dire que la chaleur émise par la source de chaleur pénètre dans la préforme de manière privilégiée en suivant la direction de chauffage. De préférence, la préforme est disposée face à une source de chaleur, de préférence posée, par une face inférieure, de préférence la face d'émail Fe, sur une plaque chauffante 22, par exemple disposée dans le four 20. L'intensité de frittage diminue donc à mesure que l'on s'éloigne de la source de chaleur, jusqu'à la face opposée, ou « face de dentine » Fd.

Lorsque la préforme est chauffée suivant une direction de chauffage, la variation de la durée du frittage additionnel permet avantageusement de modifier la profondeur de la région de la préforme qui est affectée par le frittage additionnel, mais aussi l'intensité du frittage en fonction de la profondeur.

De préférence encore, le frittage additionnel est effectué immédiatement après le frittage de base, sans déplacement de la préforme dans le four entre les deux frittages. De préférence, la préforme est initialement posée, par sa face d'émail, sur la plaque chauffante 22 disposée dans le four 20. Pour le frittage de base, l'intérieur du four est chauffé, la plaque chauffante étant éteinte. Le chauffage est alors sensiblement homogène. La plaque chauffante est alors allumée pour assurer un frittage additionnel de la région d'émail.

Le frittage additionnel peut être effectué sans éteindre le four ou après avoir éteint le four.

Le frittage additionnel peut être effectué, alors que le frittage de base se poursuit ou après achèvement dudit frittage de base, pour une partie de la préforme.

De préférence, le frittage additionnel est effectué alors que le frittage de base se poursuit pour une partie de la préforme.

En cas d'utilisation d'une plaque chauffante, comme décrit ci-dessus, de préférence, la face d'émail est chauffée par la plaque chauffante, tandis que les autres faces restent à la température de frittage du frittage de base. Après allumage de la plaque chauffante, le frittage homogène ne se poursuit donc plus que pour une partie de la préforme.

Le frittage additionnel vient donc renforcer localement le frittage de base. De préférence, le frittage additionnel se traduit alors par une augmentation locale de la température supérieure à 30°C, de préférence supérieure à 50°C, voire supérieure à 100°C, supérieure à 150°C ou supérieure à 200°C, de préférence pendant une durée supérieure à 10 min, de préférence supérieure à 15 min, supérieure à 30 min, supérieure à 60 min.

Comme représenté sur la figure 4, la direction de chauffage est de préférence sensiblement parallèle à l'axe de variation X de la préforme, de préférence de manière que la région comportant, en moyenne, les particules les plus fines soit la plus chauffée. De préférence, cette région correspond à une région d'émail de la préforme.

La deuxième courbe de la figure 3 représente la variation de l'intensité de frittage *If* résultant du frittage additionnel, en fonction de la profondeur x, à partir de la face d'émail Fe de la préforme posée sur la plaque chauffante 22, le long de la direction de chauffage. De préférence, l'intensité de frittage diminue jusqu'à une profondeur p, puis reste sensiblement constante.

Après le frittage additionnel, la région poreuse Rp présente ainsi une densité et une dureté supérieures à celles de la région très poreuse Rpp (Figure 4).

De préférence, la région poreuse présente une porosité ouverte inférieure de 1%, de préférence inférieure de 5%, de préférence inférieure de 10% à la région très poreuse.

De préférence, toute région du support présente une porosité ouverte supérieure à 10%, mesurée selon la norme ISO 5017, ce qui facilite l'infiltration de la résine.

De préférence encore, toute région du support présente une porosité ouverte comprise entre 25% et 50 %. Le plus grand diamètre des pores ouverts est de préférence compris entre 0,1 µm et 1 µm.

### Support poreux

L'invention concerne également un support poreux obtenu par frittage d'une préforme selon l'invention.

La taille moyenne de pores dans la région poreuse est de préférence supérieure à 0,1 µm et inférieure à 0,3 µm.

La taille moyenne de pores dans la région très poreuse est de préférence supérieure à la taille moyenne de pores dans la région poreuse. Elle est de préférence supérieure à 0,2 µm et inférieure à 0,3 µm.

Les régions poreuse et très poreuse sont de préférence à deux extrémités opposées du support.

La région poreuse peut être incluse dans une région d'émail ou réciproquement. De préférence, la région poreuse est sensiblement confondue avec une région d'émail.

La région très poreuse peut être incluse dans une région de dentine ou réciproquement. De préférence, la région très poreuse est sensiblement confondue avec une région de dentine.

La correspondance entre la région poreuse et la région d'émail est en particulier possible lorsque la préforme est posée une plaque chauffante par une face correspondant à la face intérieure de la suspension lors de sa centrifugation, c'est-à-dire à la face la plus proche du centre de rotation lors de la centrifugation.

La correspondance entre la région très poreuse et la région de dentine est également possible lorsque la préforme est posée une plaque chauffante par une face Fe correspondant à la face intérieure de la suspension lors de sa centrifugation, c'est-à-dire à la face la plus proche du centre de rotation lors de la centrifugation.

L'invention n'est pas limitée par la nature chimique ou la forme générale du support, pourvu que le support soit suffisamment poreux et comporte des pores ouverts interconnectés, y compris dans sa masse.

De préférence encore, avant toute infiltration de résine, le support poreux présente, en moyenne, une densité supérieure à 60 % et/ou inférieure à 85%.

Dans un mode de réalisation, la préforme et/ou le support présentent la forme générale d'une dent ou d'un bloc parallélépipédique de dimensions sensiblement équivalentes à celles d'une dent, par exemple dont la plus grande dimension est inférieure à 2 cm, de préférence inférieure à 11,5 cm et/ou la plus petite dimension est supérieure à 5 mm.

La préforme et/ou le support peuvent également se présenter sous la forme d'un disque, par exemple de 100 mm de diamètre, de préférence présentant une épaisseur supérieure à 10 mm et/ou inférieure à 25 mm, par exemple de 12 mm, 14 mm ou 20 mm. La préforme et/ou le support doivent alors être découpés pour former de blocs de dimensions sensiblement équivalentes à celles d'une dent.

Le support poreux est de préférence en un matériau céramique fritté, de préférence choisi en un ou plusieurs oxydes métalliques, sous forme de vitrocéramique, de verre, ou de céramique cristalline telle que le quartz, l'alumine ou la mullite.

### Procédé de fabrication d'un bloc composite à partir du support poreux

L'invention concerne également un procédé de fabrication d'un bloc composite, comportant les étapes A) à D) de manière à fabriquer support poreux selon l'invention, puis des étapes E) et F) suivantes :
E) infiltration du support poreux au moyen d'une résine à l'état liquide ;
F) durcissement de la résine à l'état liquide imprégnant le support.

Le bloc composite résulte du durcissement de la résine infiltrée.

Les techniques d'infiltration connues peuvent être utilisées.

La résine peut être un monomère ou un mélange de monomères. De préférence, la résine à l'état liquide ne comporte qu'un mélange de monomères et un catalyseur.

De préférence, le bloc composite est constitué par le support imprégné de la résine durcie, une unique résine ayant été infiltrée dans le support. La variation dans la distribution des tailles de particules dans le support permet en effet d'obtenir les variations des propriétés mécaniques et optiques souhaitées, sans qu'il soit nécessaire de faire varier la nature de la résine en fonction de la région du support. La fabrication du bloc composite en est considérablement simplifiée.

De préférence, la résine est chemo-polymérisable, thermopolymérisable, ou thermoplastique.

Dans un mode de réalisation préféré, la résine à l'état liquide ne comporte pas sensiblement pas de particules.

Dans un mode de réalisation, la résine à l'état liquide ne comporte pas de pigment (particule de taille nanométrique), voire pas de particule solide.

Pour faciliter la pénétration de la résine à l'état liquide, il est en effet préférable qu'elle présente une viscosité faible. En particulier, elle ne devrait pas présenter une nature pâteuse. Le cas échéant, la viscosité peut être réduite par un chauffage modéré.

La nature de la résine n'est pas limitative.

La résine peut être en particulier choisie parmi les résines polymérisables décrites dans US 5,869,548, US 5,843,348 et EP 0 701 808.

De préférence, la résine est choisie dans la liste suivante :
- une résine monomère chemopolymérisable ou thermopolymérisable, de préférence une résine vinylester ou acrylique. La résine peut en particulier être choisie dans le groupe formé par le 2-Hydroxyethyl methacrylate, CAS 868-77-9 (HEMA), le Tetraethylene glycol dimethacrylate CAS 109-17-1 (TEGDMA), le 2,2-bis-(4-(2-hydroxy-3-méthacryloyloxy-propoxy)phenyl)propane, CAS 1565-94-2 (BIS-GMA), l'urethane dimethacrylate 1,6-bis(methacryloxy-2ethoxycarbonylamino)-2,4,4-trimethylhexane, (UDMA) CAS 72869-86-4, l'éthylène-glycol-diméthacrylate (EGDMA), le diéthylène-glycol-diméthacrylate (DEGDMA), le Bisphenol A-dimethacrylate, CAS 109-17-1 (BADMA) ;
- une résine thermoplastique, en particulier choisie parmi les polyesters saturés, et notamment le Polyethylene Terephtalate (PET) et le Poly(1,4-butylene terephtalate), CAS 24968-12-5 (PBT), les Polycarbonates Poly(bisphenol A carbonate), CAS 25037-45-0 (PC), le bisphénol A-carbonate, et les polyamides.

Pour catalyser les matériaux d'imprégnation chemopolymérisables, il est possible d'utiliser des peroxydes, et en particulier le Dibenzoylperoxyd, CAS 94-36-0, le Methyl Ethyl Ketone Peroxides, CAS 1338-23-4, le Di-tert-amyl peroxide, CAS 10508-09-5, le Di-tert.-butylperoxide, CAS 110-05-4, ou le Cumene Hydroperoxide, CAS 80-15-9.

Pour accélérer le durcissement avec du Dibenzoylperoxyd, CAS 94-36-0, il est possible d'utiliser de la diméthyl-aniline (DMA), de la diéthyl-aniline (DEA) ou de la diméthyl-para-toluidine (DMPT). Pour accélérer le durcissement avec du Methyl Ethyl Ketone Peroxides, CAS 1338-23-4, il est possible d'utiliser, en particulier, du Cobalt (II) 2-ethylhexanoate.

De préférence, on crée un vide dans le support avant le début de l'infiltration de la résine à l'état liquide. Ce vide correspond de préférence à une pression inférieure à 100 mbar, de préférence inférieure à 20 mbar. Avantageusement, le vide favorise la pénétration de la résine à l'état liquide.

On appelle V_{P}(1) le volume poreux ouvert total du support poreux, avant toute infiltration de résine, mesuré à une température de 20 °C et sous une pression de 1 bar (pression ambiante).

On appelle V_{L}(1) le volume de résine à l'état liquide infiltré dans le support, mesuré à une température de 20 °C et sous une pression de 1 bar.

Dans un premier mode de réalisation, la résine à l'état liquide vient remplir le volume poreux ouvert V_{P}(1) par capillarité, à basse pression, classiquement à pression ambiante. Le volume de résine à l'état liquide infiltré dans le support, V_{L}(1), est donc sensiblement égal au volume poreux ouvert V_{P}(1).

L'infiltration de la résine à l'état liquide peut être effectuée à pression atmosphérique ou sous une pression supérieure à la pression atmosphérique. Une infiltration à pression atmosphérique est avantageusement simple à mettre en oeuvre, mais requiert l'utilisation d'une résine à l'état liquide de faible viscosité. Une infiltration sous une pression supérieure est également possible, et peut même être nécessaire si la résine à l'état liquide présente une viscosité trop élevée.

Cependant, en durcissant la résine se rétracte de sorte que le volume occupé par la résine à l'état solide V_{M}(1) est inférieur au volume poreux ouvert V_{L}(1). Par exemple, la rétraction résultant d'une polymérisation à température ambiante peut classiquement conduire à une diminution du volume occupé par la résine comprise entre 6 % et 15 % de son volume initial.

Il en résulte des contraintes élevées en traction à l'interface entre la résine et la surface du support définissant les pores, ce qui peut conduire à un décollement de la résine, et donc à une réduction de la durée de vie de la prothèse dentaire, la rendant impropre à la commercialisation.

De préférence, dans un deuxième mode de réalisation préféré, la résine imprégnant le support est durcie à l'état liquide, alors que la résine est soumise à une haute pression, supérieure à 300 bar.

De préférence, on fait pénétrer et durcir dans le support un volume de résine à l'état liquide V_{L} supérieur d'au moins 2 %, de préférence d'au moins 5 %, de préférence d'au moins 10 %, voire d'au moins 15 %, au volume V_{P} des pores ouverts du support, les volumes V_{L} et V_{P} étant mesurés à une température de 20 °C et sous une pression de 1 bar. Autrement dit, pour mesurer le volume V_{L}, on considère la résine, à l'état liquide, qui a été infiltrée dans les pores du support sous haute pression (dont le volume, au moment de cette infiltration, correspond sensiblement au volume V_{P} des pores ouverts du support), mais en considérant le volume occupé par cette résine, à l'état liquide, à une température de 20 °C et à une pression de 1 bar, c'est-à-dire avant qu'elle ne soit mise sous pression.

La résine à l'état liquide infiltrée peut en particulier être soumise à une pression supérieure à 400 bar, de préférence supérieure à 500 bar, supérieure à 1000 bar, supérieure à 2000 bar, supérieure à 3000 bar, supérieure à 4000 bar, voire supérieure à 5000 bar. Ces hautes pressions augmentent les masses volumiques de la résine à l'état liquide et du matériau constituant le support. Cependant, la compressibilité de la résine à l'état liquide est supérieure à celle du matériau constituant le support. La quantité de résine à l'état liquide qu'il est possible d'infiltrer, par unité de volume des pores ouverts, est donc supérieure à celle qu'il serait possible d'infiltrer en n'appliquant que des pressions plus faibles, et en particulier la pression atmosphérique.

La mise sous haute pression conduit à une réduction du volume de la résine, ce qui permet d'obtenir un volume de résine à l'état liquide V_{L} supérieur d'au moins 2 %, de préférence d'au moins 5 %, de préférence d'au moins 10 %, voire d'au moins 15 %, au volume V_{P} des pores ouverts du support.

En faisant durcir sous pression la résine à l'état liquide, il devient ainsi possible, après retour à la pression atmosphérique, de créer des précontraintes tendant à comprimer la résine. Il en résulte une résistance mécanique considérablement améliorée.

La haute pression doit être exercée sur la résine infiltrée dans le support tandis qu'elle est encore à l'état liquide et jusqu'à ce qu'elle ait, au moins partiellement durci. De préférence, la totalité de la résine à l'état liquide imprégnant le support est durcie avant retour à la pression atmosphérique. De préférence, la pression est maintenue sensiblement constante jusqu'à ce que la totalité de la résine infiltrée ait durci.

La haute pression peut également être exercée pendant tout ou partie de la phase d'infiltration, ce qui, avantageusement, facilite la pénétration de la résine à l'état liquide, et autorise donc l'utilisation de résines plus visqueuses.

De préférence, la haute pression est exercée de manière isostatique, ou "uniaxiale". Tous les procédés connus de mise sous pression peuvent être utilisés.

Avantageusement, dans le deuxième mode de réalisation préféré, le bloc composite obtenu ne présente alors pas de contraintes mécaniques tendant à décoller la résine du support (contraintes de traction sur la résine). Au contraire, de préférence, le "survolume" de résine infiltré et durci est déterminé, en fonction de la résine et du support, pour créer une précontrainte, c'est-à-dire une pression permanente entre résine à l'état solide et le support. Autrement dit, de préférence, la résine à l'état solide est comprimée par le support fritté. La résistance mécanique du bloc composite en est considérablement augmentée.

Alternativement à la mise sous haute pression décrite ci-dessus ou, de préférence, en complément à cette mise sous haute pression, on poursuit l'infiltration de résine à l'état liquide, le cas échéant sous pression, pendant le durcissement de la résine déjà infiltrée et, de préférence encore, on contrôle ce durcissement de manière qu'il s'effectue depuis l'intérieur du support vers sa périphérie. Avantageusement, la résine durcie ne s'oppose donc pas à la pénétration de résine à l'état liquide supplémentaire au sein du support. Il est ainsi possible de compenser la diminution du volume occupé par la résine infiltrée du fait de son durcissement, et au-delà, de mettre en compression la résine à l'état solide.

Pour contrôler le durcissement, il est notamment possible d'agir sur un ou plusieurs des paramètres suivants :
- la concentration d'accélérateur et/ou de catalyseur dans la résine à l'état liquide ;
- la température et la durée de maintien à cette température ;
- la nature chimique de la résine.

De préférence, les conditions optimales, et en particulier la haute pression éventuellement mise en oeuvre à l'étape F) et éventuellement à l'étape E), sont déterminées, en fonction de la résine et du support, par des mesures de l'homogénéité de la microdureté, de la résistance mécanique et des propriétés optiques.

La résine peut en particulier être une résine chemopolymérisable, classiquement mélangée à un catalyseur et à un accélérateur, infiltrée par exemple avec une pression de 500 bar et à une température comprise entre 80°C et 100°C.

La résine peut également être une résine thermoplastique infiltrée par exemple sous une pression isostatique de 2500 bar, à 250°C, dans un support préalablement mis sous vide et chauffé à 250°C. La résine thermoplastique peut être également infiltrée, par exemple, à une pression de 3500 bar, à une température de 300°C, dans un support préalablement mis sous vide et chauffé à une température de 300°C.

Par exemple, lorsque la résine est chemo-polymérisable, il est possible d'y ajouter une quantité d'accélérateur variable. Au début de l'infiltration, par exemple, on peut infiltrer une résine comportant une quantité élevée d'accélérateur, puis, au fur et à mesure de l'infiltration, diminuer la concentration d'accélérateur dans la résine infiltrée.

La nature de la résine peut également être variable. Par exemple, il est possible, en début d'infiltration, d'infiltrer une première résine thermopolymérisable à une première température, puis d'infiltrer une deuxième résine thermopolymérisable à une deuxième température supérieure à la première température. Par exemple, l'infiltration peut commencer avec du peroxyde de benzoyle, polymérisable à 80 °C, puis se poursuivre avec du di-t-butyl-1,2,1-peroxyde ou du di-t-amyl-1,4,2-peroxyde ou encore du comyl-1,8,8-peroxyde, polymérisables à 120 °C. Pour contrôler le durcissement, il suffit alors de chauffer le support à une température comprise entre 80 °C et 120 °C, par exemple 90°C, afin de ne durcir que la première résine, au coeur du support, puis de chauffer ce support à plus de 120 °C afin de durcir la deuxième résine à la périphérie. Une infiltration sous une pression d'environ 2000 bar est avantageuse.

En variante, il est possible d'infiltrer d'abord au coeur du support une résine chemopolymérisable, par exemple une première résine mélangée à un catalyseur et à un accélérateur, puis, à sa périphérie, une résine thermopolymérisable, par exemple une deuxième résine mélangée à un catalyseur. Le durcissement de la résine périphérique peut alors être effectué, par chauffage par exemple entre 80°C et 100°C, après durcissement de la résine disposée au coeur de du support. Une infiltration sous une pression d'environ 1500 bar est avantageuse.

Le cas échéant, le bloc composite est soumis à un traitement thermique adapté pour parfaire la polymérisation, par exemple de 100°C pendant une heure.

### Bloc composite fabriqué à partir du support poreux

L'invention concerne encore un bloc composite comportant un support selon l'invention, imprégné d'une résine à l'état solide 30 (Figure 5), de préférence fabriqué suivant un procédé de fabrication selon l'invention, et en particulier avec un durcissement, au moins d'une partie de la résine, sous haute pression (deuxième mode de réalisation préféré).

De préférence, le support est conformé de manière que le bloc composite puisse être usiné par un dispositif CAD-CAM, notamment par un dispositif d'usinage tel que le système CELAY^{®} de la société Mikrona ou CEREC 3 de la société SIRONA. Le cas échéant, le bloc composite peut intégrer un ou plusieurs organes permettant le maintien du support par de tels dispositifs.

Comme représenté sur la figure 5, le bloc composite comporte :
- une région présentant une dureté supérieure à 240 Vickers dite « région très dure » R_{tdu}, et de préférence un module élastique de Young, mesuré suivant la norme ISO 10 477, supérieur à 30 GPa, et
- une région présentant une dureté supérieure à 60 Vickers et inférieure à 180 Vickers, dite « région dure » R_{du}, et, de préférence, un module élastique de Young, mesuré suivant la norme ISO 10 477, supérieur à 15 GPa et inférieur à 30 GPa.

Chacune desdites régions dure et très dure présente un volume supérieur à 30 mm³, de préférence supérieur à 50 mm³, de préférence supérieur à 100 mm³, de préférence supérieur à 150 mm³. Lesdites régions très dure et dure présentent la forme de couches d'épaisseur supérieure à 3 mm, de préférence supérieure à 5 mm.

De préférence, la région très dure R_{tdu} présente une dureté supérieure à 250 Vickers, de préférence supérieure à 300 Vickers, de préférence supérieure à 350 Vickers, voire supérieure à 400 Vickers, et/ou de préférence inférieure à 450 Vickers.

De préférence, la région dure R_{du} présente une dureté supérieure à 70 Vickers, de préférence supérieure à 80 Vickers, et/ou inférieure à 180 Vickers, de préférence inférieure à 170 Vickers, voire inférieure à 160 Vickers, ou inférieure à 150 Vickers.

De préférence, la région très dure R_{tdu} présente un module élastique de Young, mesuré suivant la norme ISO 10 477, supérieur à 35 GPa, de préférence supérieur à 40 GPa, et/ou de préférence inférieur à 60 GPa.

De préférence, la région dure R_{du} présente un module élastique de Young, mesuré suivant la norme ISO 10 477, supérieur à 16 GPa, de préférence supérieur à 18 GPa, et/ou inférieur à 28 GPa, de préférence inférieur à 25 GPa.

Avantageusement, ces propriétés mécaniques confèrent une longue durée de vie à la prothèse dentaire obtenue à partir de ce bloc composite.

La région très dure peut être incluse dans une région poreuse ou réciproquement. De préférence, la région très dure est sensiblement confondue avec une région poreuse.

La région dure peut être incluse dans une région très poreuse ou réciproquement. De préférence, la région dure est sensiblement confondue avec une région très poreuse.

### Procédé de fabrication d'une prothèse dentaire

Il est également décrit un procédé de fabrication d'une prothèse dentaire comprenant une opération d'usinage d'un bloc composite selon l'invention, ainsi qu'une prothèse dentaire fabriquée ou susceptible d'avoir été fabriquée suivant un procédé conforme à l'invention.

Un procédé de fabrication d'une prothèse dentaire composite peut comprendre les étapes suivantes :
1) préparation d'un support poreux selon l'invention ;
2) optionnellement, rectification de la forme du support poreux ;
3) optionnellement, traitement de silanage à la surface des pores ouverts du support poreux ;
4) imprégnation du support poreux avec une résine à l'état liquide ;
5) durcissement de la résine à l'état liquide infiltrée dans le support poreux, optionnellement suivi d'un traitement thermique de consolidation ;
6) mise en forme finale.

Le traitement de silanage, à l'étape 3), est destiné à augmenter la mouillabilité de la surface des pores par la résine à l'état liquide, et en particulier destiné à rendre cette surface plus hydrophobe. De préférence, ce traitement de silanage comprend une silanisation au moyen d'alcoxysilane, d'halosilane, de préférence de 3-méthacryloxypropyltriméthoxysilane. Après application de l'agent de silanage, le support est séché, de préférence à une température comprise entre 100 °C et 200 °C, classiquement pendant plusieurs heures.

Le traitement de silanage peut par exemple être effectué conformément au procédé décrit dans US 5,869,548.

Les étapes 4) et 5) correspondent aux étapes E) et F) d'un procédé de fabrication d'un bloc composite selon l'invention, tel que décrit précédemment.

### Exemples

L'exemple suivant est fourni à des fins illustratives et non limitatives.

Les poudres suivantes ont été mélangées :
- 30% en volume une poudre de particules d'alumine présentant des percentiles D₁₀ de 0,5 µm, D₅₀ de 3 µm et D₉₀ de 6 µm, apportant notamment des particules d'émail ; et
- 70% en volume d'une poudre de particules d'alumine présentant des percentiles D₁₀ de 2 µm, D₅₀ de 5,5 µm et D₉₀ de 8 µm, apportant notamment des particules de dentine ;

L'ensemble de particules ainsi formé a été mélangé avec de l'eau, de manière à constituer une suspension. La matière sèche (particules d'alumine) représentait 50% du volume de la suspension.

0,05% acide citrique (liant), en pourcentage de la masse de ladite suspension, ont été ajoutés.

L'ensemble a été mélangé au moyen d'un mélangeur planétaire, puis centrifugé de manière à appliquer une accélération de 150 G pendant 30 minutes. La masse centrifugée présentait les dimensions suivantes : L : 40 mm ; 1 : 20 mm ; e : 16 mm.

Le surnageant a été jeté.

Le rapport Ve/(Ve+Vd) à proximité de la face de la masse centrifugée proche de l'axe de rotation (face d'émail) était de 0,9.

La région de la préforme s'étendant depuis la face d'émail jusqu'à un plan parallèle à la face d'émail et défini pour que ladite région représente 25% du volume du support, constituait une « région d'émail ».

Le rapport Ve/(Ve+Vd) à proximité de la face opposée (face de dentine), éloignée de l'axe de rotation était de 0,1.

La région de la préforme s'étendant depuis la face de dentine jusqu'à un plan parallèle à la face de dentine et défini pour que ladite région représente 25% du volume du support, constituait une « région de dentine ».

La masse centrifugée a ensuite été séchée à 20°C pendant 24 heures, ce qui a conduit à une préforme.

La préforme a été démoulée, puis introduite dans un four, la face d'émail étant posée sur une plaque chauffante préalablement disposée dans le four.

Le four a été porté à 1100°C pendant 4 heures, afin d'assurer un frittage de base sensiblement homogène de la préforme.

La plaque chauffante sur laquelle est posée la face d'émail a ensuite été allumée, en maintenant le four à 1100°C, de manière que la face d'émail soit portée à une température de 1300°C pendant 1,5 heure, et subisse ainsi un frittage additionnel.

Le traitement thermique a conduit à un support poreux présentant une porosité ouverte moyenne de 40%.

La région du support poreux s'étendant depuis la face d'émail jusqu'à un plan parallèle à la face d'émail et défini pour que ladite région représente 25% du volume du support, présentait une taille moyenne des pores, mesurée avec un porosimètre à mercure, de 0,2 µm. Elle constituait une « région poreuse».

La région du support poreux s'étendant depuis la face de dentine jusqu'à un plan parallèle à la face de dentine et défini pour que ladite région représente 25% du volume du support, présentait une taille moyenne de pores de 0,3 µm. Elle constituait une « région très poreuse ».

Le support poreux a subi successivement
- un traitement de silanage par une solution présentant la composition suivante, en pourcentages massiques :

| | |
|---|---|
| méthoxypropanol : | 93,8 % |
| eau : | 5 % |
| acide acétique : | 0,2 % |
| silane : | 1 % |

- un séchage à 150 °C pendant 4 heures ;
- une mise sous vide ;
- une infiltration d'une résine à une température de 60°C sous une pression de 80 bar, pendant 4 heures, la composition massique de cette résine étant la suivante :

| | |
|---|---|
| UDMA : | 99 % |
| DI-TERT-AMYL -PEROXIDE : | 1 % |

- un chauffage à 150°C sous une pression de 2000 bar, pendant 1 heure afin de polymériser la résine.

Dans la pratique, il est possible, après l'opération de silanage, de disposer le support poreux dans un moule en latex, d'y faire le vide, par exemple jusqu'à une pression d'environ 100 mbar, puis d'y introduire la résine à l'état liquide sous vide et de fermer le moule. Après infiltration, le moule ainsi fermé peut alors être introduit dans un pot ou dans un autoclave où il est progressivement soumis à la pression de 2000 bar et au chauffage de consolidation de la résine, avant refroidissement et retour à la pression atmosphérique.

La région poreuse du support correspondait à une « région très dure » présentant une dureté de 380 Vickers et un module élastique de Young de 55 GPa.

La région très poreuse du support correspondait à une « région dure » présentant une dureté de 160 Vickers et un module élastique de Young de 25 GPa.

Comme cela apparaît clairement à présent, l'invention permet d'obtenir une variation continue et progressive des propriétés optiques et/ou mécaniques d'un bloc composite destiné à la fabrication d'une prothèse dentaire. Elle permet donc de fabriquer une prothèse dentaire dont l'apparence et les propriétés mécaniques sont sensiblement identiques à celles d'une dent naturelle.

Bien entendu, l'invention n'est pas limitée aux modes de réalisation décrits, ni aux exemples. En particulier, l'infiltration de résine à l'état liquide peut être limitée à une région limitée du support, et en particulier être limitée à sa région périphérique.

## Revendications

1. Préforme destinée à la fabrication d'une prothèse dentaire, ladite préforme comportant un ensemble de particules agglomérées, en matière céramique, en matière vitrocéramique ou en verre, tel que, en pourcentages volumiques :
- plus de 40% et moins de 90% des particules dudit ensemble présentent une taille supérieure à 0,5 µm et inférieure à 3,5 µm, lesdites particules étant désignées ci-après « particules d'émail », et
- plus de 10% et moins de 60% des particules dudit ensemble présentent une taille supérieure à 3,5 µm et inférieure à 5,5 µm, lesdites particules étant désignées ci-après « particules de dentine »,
la microstructure de la préforme étant telle qu'il existe un axe X, dit « axe de variation », suivant lequel le rapport Ve/(Ve+Vd) évolue de manière continue , Ve et Vd désignant les pourcentages volumiques en particules d'émail et en particules de dentine, respectivement,
les particules d'émail et de dentine représentant, ensemble, plus de 90% du volume des particules agglomérées,
la distribution granulométrique de l'ensemble des particules étant bimodale et comportant des premier et deuxième modes principaux, le premier mode principal étant supérieur à 1,5 µm et inférieur à 3,5 µm, le deuxième mode principal étant supérieur à 3,5 µm et inférieur à 5,5 µm.

2. Préforme selon la revendication immédiatement précédente, dans laquelle les particules d'émail présentent une taille moyenne D₅₀ supérieure à 1,5 µm et inférieure à 3,0 µm, et/ou les particules de dentine présentent une taille moyenne D₅₀ supérieure à 4,0 µm et inférieure à 5,0 µm.

3. Préforme selon l'une quelconque des revendications précédentes, dans laquelle, en pourcentages volumiques, plus de 50% des particules dudit ensemble sont des particules d'émail, et/ou plus de 30% des particules dudit ensemble sont des particules de dentine.

4. Préforme selon l'une quelconque des revendications précédentes, dans laquelle, suivant l'axe de variation, le pourcentage volumique de particules d'émail varie de manière inverse opposée au pourcentage volumique de particules de dentine.

5. Préforme selon l'une quelconque des revendications précédentes, dans laquelle la préforme présente une première région, dite « région d'émail », dans laquelle le rapport Ve/(Ve+Vd) est supérieur à 0 ,9 et une deuxième région, dite « région de dentine », dans laquelle le rapport Ve/(Ve+Vd) est inférieur à 0,1,
les régions d'émail et de dentine étant sous la forme de couches et s'étendant à partir de faces d'émail (Fe) et de dentine (Fd) opposées de la préforme, en considérant l'axe de variation.

6. Préforme selon la revendication immédiatement précédente, ladite région de dentine présentant un rapport Ve/(Ve+Vd) inférieur à 0,05, et/ou les particules d'émail et de dentine représentant ensemble plus de 95% du volume de la masse dudit ensemble de particules.

7. Procédé de fabrication d'une préforme selon l'une quelconque des revendications précédentes, ledit procédé comportant les étapes suivantes :
A) préparation d'une suspension comportant
- un ensemble de particules, de préférence en matière céramique, ledit ensemble comportant, en pourcentages volumiques sur la base du volume de la masse dudit ensemble de particules :
- plus de 30% et moins de 70% de particules d'émail, et
- plus de 30% et moins de 70% de particules de dentine,
- un solvant ;
B) modification de la distribution spatiale des particules de la suspension;
C) consolidation, de manière à former une préforme.

8. Procédé selon la revendication immédiatement précédente, dans lequel l'étape B) comporte une centrifugation de la suspension.

9. Procédé selon la revendication immédiatement précédente, dans lequel ladite centrifugation produit une accélération supérieure à 50 G.

10. Procédé de fabrication d'un support poreux, ledit procédé comportant une fabrication d'une préforme selon un procédé conforme à l'une quelconque des trois revendications immédiatement précédentes, puis une étape D) de frittage de ladite préforme, procédé dans lequel l'intensité du frittage d'une région de la préforme est variable en fonction de sa position dans la préforme, de préférence en fonction de sa position le long de l'axe de variation.

11. Procédé selon la revendication immédiatement précédente, comportant
- un frittage de base lors duquel toute la surface extérieure de la préforme reçoit sensiblement la même densité de flux calorifique; et
- un frittage additionnel lors duquel la densité de flux thermique est variable en fonction de la partie de la surface extérieure de la préforme considérée, la densité de flux thermique lors du frittage additionnel étant d'autant plus élevée que le rapport Ve/(Ve+Vd) est élevé.

12. Procédé selon la revendication immédiatement précédente, dans lequel, lors du frittage additionnel, une face de la préforme à proximité de laquelle la concentration en particules d'émail est la plus élevée, dite « face d'émail » (Fe), repose sur une plaque chauffante.

13. Support fabriqué suivant un procédé selon l'une quelconque des trois revendications immédiatement précédentes, ledit support comportant une région poreuse (Rp) et une région très poreuse (Rpp),
lesdites régions poreuse et très poreuse présentant chacune une épaisseur supérieure à 1 mm,
la région poreuse présentant une porosité ouverte comprise entre 5% à 20%, et
la région très poreuse présentant une porosité ouverte supérieure à 20% et inférieure à 40%, la région poreuse présentant une porosité ouverte inférieure de 5% à la région très poreuse,
lesdites régions poreuse et très poreuse présentant chacune un volume supérieur à 30 mm³.

14. Procédé de fabrication d'un bloc composite à partir d'un support poreux, ledit procédé comportant une fabrication d'un support poreux selon un procédé conforme à l'une quelconque des revendications 10 à 12, puis des étapes E) et F) suivantes :
E) infiltration du support poreux au moyen d'une résine à l'état liquide ;
F) durcissement de toute la résine à l'état liquide imprégnant le support ;
les étapes E) et F) étant effectuées sous une pression supérieure à 100 MPa ou 1000 bar.

15. Bloc composite destiné à la fabrication d'une prothèse dentaire, ledit bloc, fabriqué suivant un procédé selon la revendication 14, comportant
- une région dite « très dure » présentant une dureté supérieure à 240 Vickers, et de préférence un module élastique de Young, mesuré suivant la norme ISO 10 477, supérieur à 30 GPa, et
- une région dite « dure » présentant une dureté supérieure à 60 Vickers et inférieure à 180 Vickers, et, de préférence, un module élastique de Young, mesuré suivant la norme ISO 10 477, supérieur à 15 GPa et inférieur à 30 GPa,
chacune desdites régions dure et très dure présentant un volume supérieur à 30 mm³, lesdites régions très dure et dure présentant la forme de couches d'épaisseur supérieure 3 mm.

## Patentansprüche

1. Vorformling zur Herstellung eines Zahnersatzteils, wobei der Vorformling einen Satz von agglomerierten Partikeln aus einem keramischen Material, einem Glaskeramikmaterial oder einem Glas umfasst, sodass, in Volumenprozent:
- mehr als 40 % und weniger als 90 % der Partikel des Satzes eine Größe von mehr als 0,5 µm und weniger als 3,5 µm aufweisen, wobei die Partikel nachfolgend als "Emaillepartikel" bezeichnet werden, und
- mehr als 10 % und weniger als 60 % der Partikel des Satzes eine Größe von mehr als 3,5 µm und weniger als 5,5 µm aufweisen, wobei die Partikel nachfolgend als "Dentinpartikel" bezeichnet werden,
wobei die Mikrostruktur des Vorformlings so ist, dass eine X-Achse existiert, die als "Variationsachse" bezeichnet wird, entlang der sich das Verhältnis Ve/(Ve+Vd) kontinuierlich ändert, wobei Ve bzw. Vd die Volumenprozentwerte von Emaillepartikeln bzw. Dentinpartikeln bezeichnen,
wobei die Emaille- und Dentinpartikel zusammen mehr als 90 % des Volumens der agglomerierten Partikel darstellen, wobei die Partikelgrößenverteilung des Partikelsatzes bimodal ist und einen ersten und einen zweiten Hauptmodus umfasst, der erste Hauptmodus größer als 1,5 µm und kleiner als 3,5 µm ist, der zweite Hauptmodus größer als 3,5 µm und kleiner als 5,5 µm ist.

2. Vorformling nach dem unmittelbar vorhergehenden Anspruch, wobei die Emaillepartikel eine mittlere Größe D₅₀ von mehr als 1,5 µm und weniger als 3,0 µm aufweisen und/oder die Dentinpartikel eine mittlere Größe D₅₀ von mehr als 4,0 µm und weniger als 5,0 µm aufweisen.

3. Vorformling nach einem der vorhergehenden Ansprüche, wobei es sich, in Volumenprozent, bei mehr als 50 % der Partikel des Satzes um Emaillepartikel handelt und/oder bei mehr als 30 % der Partikel des Satzes um Dentinpartikel handelt.

4. Vorformling nach einem der vorhergehenden Ansprüche, wobei sich der Volumenprozentwert der Emaillepartikel entlang der Variationsachse entgegengesetzt zum Volumenprozentwert der Dentinpartikel ändert.

5. Vorformling nach einem der vorhergehenden Ansprüche, wobei der Vorformling einen als "Emaillebereich" bezeichneten ersten Bereich, in dem das Verhältnis Ve/(Ve+Vd) größer als 0,9 ist, und einen als "Dentinbereich" bezeichneten zweiten Bereich aufweist, in dem das Verhältnis Ve/(Ve+Vd) kleiner als 0,1 ist, wobei der Emaille- und der Dentinbereich in Form von Schichten vorliegen und sich von der Emaille- (Fe) und der Dentinstirnfläche (Fd) des Vorformlings erstrecken, die in Bezug auf die Variationsachse einander gegenüberliegen.

6. Vorformling nach dem unmittelbar vorhergehenden Anspruch, wobei der Dentinbereich ein Verhältnis Ve/(Ve+Vd) kleiner 0,05 aufweist und/oder die Emaille- und die Dentinpartikel zusammen mehr als 95 % des Volumens der Masse des Partikelsatzes darstellen.

7. Verfahren zur Herstellung eines Vorformlings nach einem der vorhergehenden Ansprüche, wobei das Verfahren die folgenden Schritte umfasst:
A) die Herstellung einer Suspension, die Folgendes umfasst:
- eine Gruppe von Partikeln, vorzugsweise aus einem keramischen Material, wobei die Gruppe in Volumenprozent, bezogen auf das Volumen der Masse der Gruppe von Partikeln, Folgendes umfasst:
- mehr als 30 % und weniger als 70 % Emaillepartikel und
- mehr als 30 % und weniger als 70 % Dentinpartikel,
- ein Lösungsmittel;
B) eine Modifikation der räumlichen Verteilung der Partikel der Suspension;
C) eine Verfestigung, um einen Vorformling zu bilden.

8. Verfahren nach dem unmittelbar vorhergehenden Anspruch, wobei Schritt B) eine Zentrifugation der Suspension umfasst.

9. Verfahren nach dem unmittelbar vorhergehenden Anspruch, wobei die Zentrifugation eine Beschleunigung von mehr als 50 G erzeugt.

10. Verfahren zur Herstellung eines porösen Trägers, wobei das Verfahren eine Herstellung eines Vorformlings gemäß einem Verfahren nach einem der drei unmittelbar vorhergehenden Ansprüche, dann einen Schritt D) eines Sinterns des Vorformlings umfasst, wobei die Intensität des Sinterns eines Bereichs des Vorformlings beim Verfahren in Abhängigkeit von seiner Position im Vorformling, vorzugsweise in Abhängigkeit von seiner Position entlang der Variationsachse, variabel ist.

11. Verfahren nach dem unmittelbar vorhergehenden Anspruch, das Folgendes umfasst:
- ein Hauptsintern, bei dem die gesamte Außenfläche des Vorformlings im Wesentlichen dieselbe Wärmeflussdichte erhält; und
- ein zusätzliches Sintern, bei dem die Wärmeflussdichte in Abhängigkeit vom Teil der Außenfläche des betrachteten Vorformlings variabel ist, wobei die Wärmeflussdichte beim zusätzlichen Sintern umso höher ist, je größer das Verhältnis Ve/(Ve+Vd) ist.

12. Verfahren nach dem unmittelbar vorhergehenden Anspruch, wobei beim zusätzlichen Sintern eine als "Emaillestirnfläche" (Fe) bezeichnete Stirnfläche des Vorformlings, in deren Nähe die Konzentration von Emaillepartikeln am höchsten ist, auf einer Heizplatte ruht.

13. Träger, der gemäß einem Verfahren nach einem der drei unmittelbar vorhergehenden Ansprüche hergestellt wird, wobei der Träger einen porösen Bereich (Rp) und einen sehr porösen Bereich (Rpp) umfasst,
wobei der poröse und der sehr poröse Bereich jeweils eine Dicke von mehr als 1 mm aufweisen,
wobei der poröse Bereich eine offene Porosität zwischen 5 % und 20 % aufweist und
wobei der sehr poröse Bereich eine offene Porosität von mehr als 20 % und weniger als 40 % aufweist, wobei der poröse Bereich eine offene Porosität aufweist, die um 5 % geringer als diejenige des sehr porösen Bereichs ist,
wobei der poröse und der sehr poröse Bereich jeweils ein Volumen von mehr als 30 mm³ aufweisen.

14. Verfahren zur Herstellung eines Verbundstoffblocks aus einem porösen Träger, wobei das Verfahren eine Herstellung eines porösen Trägers gemäß einem Verfahren nach einem der Ansprüche 10 bis 12, dann die folgenden Schritte E) und F) umfasst:
E) das Infiltrieren des porösen Trägers mit einem Harz im flüssigen Zustand;
F) das Härten des gesamten Harzes im flüssigen Zustand, mit dem der Träger getränkt ist;
wobei die Schritte E) und F) unter einem Druck vom mehr als 100 MPa oder 1000 bar durchgeführt werden.

15. Verbundstoffblock, der zur Herstellung eines Zahnersatzteils vorgesehen ist, wobei der gemäß einem Verfahren nach Anspruch 14 hergestellte Block Folgendes umfasst:
- einen als "sehr hart" bezeichneten Bereich mit einer Härte von mehr als 240 Vickers und vorzugsweise einem gemäß der Norm ISO 10 477 gemessenen Youngschen Modul von mehr als 30 GPa und
- einen als "hart" bezeichneten Bereich mit einer Härte von mehr als 60 Vickers und weniger als 180 Vickers und vorzugsweise einem gemäß der Norm ISO 10 477 gemessenen Youngschen Modul von mehr als 15 GPa und weniger als 30 GPa,
wobei sowohl der harte als auch der sehr harte Bereich ein Volumen von mehr als 30 mm³ aufweisen, wobei der sehr harte und der harte Bereich eine Form von Schichten mit einer Dicke von mehr als 3 mm aufweisen.

## Claims

1. Preform intended for the production of a dental prosthesis, said preform comprising a group of agglomerated ceramic, glass-ceramic or glass particles, such that, as volume percents:
- more than 40% and less than 90% of the particles of said group have a size greater than 0.5 µm and less than 3.5 µm, said particles hereinafter being denoted "enamel particles", and
- more than 10% and less than 60% of the particles of said group have a size greater than 3.5 µm and less than 5.5 µm, said particles hereinafter being denoted "dentine particles",
the microstructure of the preform being such that there is an axis X, termed "axis of variation", along which the Ve/(Ve+Vd) ratio changes continuously, Ve and Vd denoting the volume percents of enamel particles and of dentine particles, respectively,
the enamel and dentine particles representing, together, more than 90% of the volume of the agglomerated particles,
the particle size distribution of the group of particles being bimodal and comprising first and second principal modes, the first principal mode being greater than 1.5 µm and less than 3.5 µm, the second principal mode being greater than 3.5 µm and less than 5.5 µm.

2. Preform according to the immediately preceding claim, wherein the enamel particles have a mean size D₅₀ greater than 1.5 µm and less than 3.0 µm, and/or the dentine particles have a mean size D₅₀ greater than 4.0 µm and less than 5.0 µm.

3. Preform according to either one of the preceding claims, wherein, as volume percents, more than 50% of the particles of said group are enamel particles, and/or more than 30% of the particles of said group are dentine particles.

4. Preform according to any one of the preceding claims, wherein, along the axis of variation, the volume percent of enamel particles varies inversely opposite to the volume percent of dentine particles.

5. Preform according to any one of the preceding claims, wherein the preform has a first region, termed "enamel region", in which the Ve/(Ve+Vd) ratio is greater than 0.9 and a second region, termed "dentine region", in which the Ve/(Ve+Vd) ratio is less than 0.1,
the enamel and dentine regions being in the form of layers and extending from opposite enamel (Fe) and dentine (Fd) faces of the preform, taking into consideration the axis of variation.

6. Preform according to the immediately preceding claim, said dentine region having a Ve/(Ve+Vd) ratio of less than 0.05, and/or the enamel and dentine particles representing, together, more than 95% of the volume of the mass of said group of particles.

7. Process for producing a preform according to any one of the preceding claims, said process comprising the following steps:
A) preparing a suspension comprising
- a group of particles, preferably ceramic particles, said group comprising, as volume percents on the basis of the volume of the mass of said group of particles:
- more than 30% and less than 70% of enamel particles, and
- more than 30% and less than 70% of dentine particles,
- a solvent;
B) modifying the spatial distribution of the particles of the suspension;
C) consolidating so as to form a preform.

8. Process according to the immediately preceding claim, wherein step B) comprises a centrifugation of the suspension.

9. Process according to the immediately preceding claim, wherein said centrifugation produces an acceleration of greater than 50 G.

10. Process for producing a porous support, said process comprising a production of a preform according to a process in accordance with any one of the three immediately preceding claims, then a step D) of sintering said preform, in which process the intensity of the sintering of a region of the preform is variable according to its position in the preform, preferably according to its position along the axis of variation.

11. Process according to the immediately preceding claim, comprising
- basic sintering during which the entire external surface of the preform receives substantially the same density of heat flow, and
- additional sintering during which the density of the heat flow is variable according to the part of the external surface of the preform under consideration, the density of heat flow during the additional sintering being higher the higher the Ve/(Ve+Vd) ratio.

12. Process according to the immediately preceding claim, wherein, during the additional sintering, one face of the preform in proximity to which the concentration of enamel particles is the highest, termed "enamel face" (Fe), sits on a hot plate.

13. Support produced according to a process according to any one of the three immediately preceding claims, said support comprising a porous region (Rp) and a very porous region (Rpp),
said porous and very porous regions each having a thickness of greater than 1 mm,
the porous region having an open porosity of between 5% and 20%, and
the very porous region having an open porosity of greater than 20% and less than 40%, the porous region having an open porosity which is 5% less than the very porous region,
said porous and very porous regions each having a volume of greater than 30 mm³.

14. Process for producing a composite block from a porous support, said process comprising a production of a porous support according to a process in accordance with any one of Claims 10 to 12, then steps E) and F) as follows:
E) infiltrating the porous support by means of a resin in the liquid state;
F) curing all of the resin in the liquid state impregnating the support;
steps E) and F) being carried out under a pressure of greater than 100 MPa or 1000 bar.

15. Composite block intended for the production of a dental prosthesis, said block, produced according to a process according to Claim 14, comprising
- a "very hard" region having a hardness greater than 240 Vickers, and preferably a Young's elastic modulus, measured according to ISO standard 10 477, greater than 30 GPa, and
- a "hard" region having a hardness greater than 60 Vickers and less than 180 Vickers, and, preferably, a Young's elastic modulus, measured according to ISO standard 10 477, greater than 15 GPa and less than 30 GPa,
each of said hard and very hard regions having a volume of greater than 30 mm³, said very hard and hard regions being in the form of layers with a thickness of greater than 3 mm.
